# EUROPEAN PATENT SPECIFICATION

(11) **EP 3 730 179 B1**
(45) Date of publication and mention of the grant of the patent: **12.10.2022**
(21) Application number: 20171358.3
(22) Date of filing: 24.04.2020
(51) Int. Cl.: A61M 25/09

(54) **GUIDEWIRE WITH TACTILE FEEL**
FÜHRUNGSDRAHT MIT TAKTILER HAPTIK
FIL-GUIDE À SENSATION TACTILE

(30) Priority: 25.04.2019 US 201962838355 P; 25.04.2019 US 201962838357 P
(43) Date of publication of application: 28.10.2020
(73) Proprietor: Lake Region Medical, Inc., Wilmington MA 01887 (US)
(72) Inventor: Keary, David, Galway (IE); Mc Allister, Gordon, Galway (IE); Hunt, Rhona, F42 WV50 (IE); Brogan, James, Galway (IE); Freeley, Joe, Galway (IE)
(74) Representative: J A Kemp LLP

(56) References cited:
- EP-A1- 2 140 904
- US-A1- 2004 167 439
- US-A1- 2018 193 606

## Description

### BACKGROUND

Conventional guidewires on the market typically include polytetrafluroethylene (PTFE) or other polymer layer with a smooth surface finish with no or limited tactile feel (grip) on the guidewire shaft. Grip and/or tactile feel allow better feedback to the physician during use of the guidewire.

Some conventional guidewires do include tactile feel, which is typically accomplished with the addition of a raised PTFE spiral cut layer to the guidewire surface. That is, some conventional guidewires typically accomplish tactile feel by applying spiral cut PTFE material with a raised section over an existing layer of PTFE material of the guidewire.

US 2018/1936506, US 2004/167439 and EP 2,140,904 disclose prior art guidewires.

### OVERVIEW

This overview is intended to provide an overview of subject matter of the present disclosure. It is not intended to provide an exclusive or exhaustive explanation of the invention. The detailed description is included to provide further information about the present disclosure.

The present inventors have recognized, among other things, that the present subject matter can be used to provide a guidewire with tactile feel to satisfy design requirements required by physicians by incorporating the tactile feel into the guidewire. In various examples, the present subject matter is advantageous in that it provides for a guidewire that includes a grip portion to enhance tactile feel, handling, control, and advancement recognition for the physician or other user of the guidewire. In some examples, the present invention facilitates tracking of a catheter or other indicated interventional device over the guidewire by reducing a force needed to do so.

There is disclosed subject matter including a guidewire. The guidewire includes an elongate core wire including a distal end, a proximal end, and a longitudinal axis extending along a length of the core wire from the distal end to the proximal end. A grip portion is disposed on the core wire. The grip portion is configured to provide tactile feel to a user of the guidewire.

The subject matter is optionally configured such that the grip portion is disposed proximate the proximal end of the core wire.

The subject matter is optionally configured such that the core wire includes a distal portion extending proximally from the distal end and a proximal portion extending distally from the proximal end. The distal portion may include a distal profile and the proximal portion may include a proximal profile, the distal profile being smaller than the proximal profile.

The subject matter is optionally configured such that the grip portion extends at least partially along the proximal portion of the core wire.

The subject matter is configured such that the grip portion includes a polymer tube applied to the core wire. The polymer tube includes a sidewall extending between first and second ends of the polymer tube and at least one cutout through the sidewall of the polymer tube.

The subject matter is optionally configured such that the at least one cutout includes a plurality of cutouts disposed within the sidewall of the polymer tube to form a cut pattern of the grip portion.

The subject matter is optionally configured such that the at least one cutout includes at least one laser cut cutout through the sidewall of the polymer tube.

The subject matter is optionally configured such that the polymer tube includes a heat shrinkable polymer tube. The polymer tube may include a heat shrunk polymer tube (i.e. an already shrunk tube).

The subject matter is optionally configured such that the grip portion includes a grip pattern formed in the core wire.

The subject matter is optionally configured such that the grip pattern includes at least one groove cut into the core wire, the groove extending along at least part of the grip portion of the core wire.

The subject matter is optionally configured such that the at least one groove spirally extends along the core wire.

The subject matter is optionally configured such that the grip pattern includes at least part of the grip portion of the core wire including a non-circular shape. At least the part of the grip portion of the core wire may be twisted about the longitudinal axis of the core wire to rotate the non-circular shape along at least the part of the grip portion.

There is disclosed subject matter that includes a method of making a guidewire with tactile feel. This subject matter may be combined with the other subject matter disclosed herein. The disclosed method includes forming an elongate core wire. The core wire includes a distal end, a proximal end, and a longitudinal axis extending along a length of the core wire from the distal end to the proximal end. A grip portion is formed on the core wire. The grip portion is configured to provide tactile feel to a user of the guidewire.

The subject matter is optionally configured such that forming the grip portion includes forming the grip portion on the core wire proximate the proximal end of the core wire.

The subject matter is configured such that forming the grip portion includes cutting at least one cutout through a sidewall of a polymer tube. The polymer tube includes the sidewall extending between first and second ends of the polymer tube. The polymer tube is applied to the core wire, wherein the at least one cutout forms a cut pattern of the grip portion.

The subject matter is optionally configured such that applying the polymer tube includes heat shrinking the polymer tube directly or indirectly onto the core wire.

The subject matter is optionally configured such that forming the grip portion includes forming a grip pattern in the core wire.

The subject matter is optionally configured such that forming the grip pattern in the core wire includes cutting at least one groove into the core wire. The groove may extend along at least part of the grip portion of the core wire.

The subject matter is optionally configured such that cutting the at least one groove into the core wire includes spirally cutting the at least one groove into the core wire so that the at least one groove spirally extends along the core wire.

The subject matter is optionally configured such that forming the grip pattern includes forming at least part of the grip portion of the core wire into a non-circular shape. At least the part of the grip portion of the core wire may be twisted about the longitudinal axis of the core wire to rotate the non-circular shape along at least the part of the grip portion.

### BRIEF DESCRIPTION OF THE DRAWINGS

FIG. 1 is a side view of a guidewire in accordance with the invention.
FIG. 2 is a side view of a guidewire in accordance with another example of the invention.
FIG. 3 is a side view of a guidewire in accordance with another example of the invention.
FIG. 4 is a side view of a guidewire in accordance with another example of the invention.
FIG. 5 is a side view of a guidewire in accordance with another example of the invention.
FIGS. 6A-6F are cross-sectional views of a grip portion of a guidewire in accordance with another example of the invention.
FIGS. 7A-7D are cross-sectional views of a grip portion of a guidewire in accordance with another example of the invention.
FIGS. 8A-8D are cross-sectional views of a grip portion of a guidewire in accordance with another example of the invention.
FIGS. 9A-9E are cross-sectional views of a grip portion of a guidewire in accordance with another example of the invention.
FIG. 10 shows a method of making a guidewire in accordance with the invention.
FIG. 11 shows a method of making a guidewire in accordance with another example of the invention.
FIG. 12 shows a method of making a guidewire in accordance with another example of the invention.

### DETAILED DESCRIPTION

The present invention relates generally to providing a guidewire with tactile feel to satisfy design requirements required by physicians by incorporating the tactile feel into the guidewire. More specifically, the present invention relates to a guidewire and a method therefor, wherein the guidewire includes a grip portion to enhance tactile feel, handling, control, and advancement recognition for the physician or other user of the guidewire. In some examples, the grip portion of the present invention results in less contact area with the catheter wall, such that the force to track a catheter or other indicated interventional device over the guidewire can be reduced. In some examples, the present invention allows a core wire of the guidewire to be coated after forming the grip portion in the core wire, for instance, to enhance efficient catheter/device exchanges.

In some examples, the purpose of the present inventive subject matter is to provide a guidewire with tactile feel to satisfy design requirements required by physicians by incorporating the tactile feel into the guidewire. In some examples, this is accomplished by shrinking a polymer tube with a cut pattern or design onto the core wire. In other examples, this is accomplished by drawing a shape through a die and twisting the core wire at the same time to form a cylindrical shape with a twist. In other examples, this is accomplished by machining or otherwise forming a shape in the core wire and twisting the core wire to form a cylindrical shape with a twist. In other examples, this is accomplished by machining or otherwise forming a twisting shape in the core wire to form a cylindrical shape with a twist. In still other examples, this is accomplished by forming one or more substantially helical or spirally-extending grooves into and along the core wire.

In some examples, by shrinking a polymer tube with a cut pattern onto an existing core wire the tactile feel/bumpy surface can be incorporated into existing guidewire designs. In some examples, the polymer tube can be coated, for instance, to either increase the lubricity or increase the friction of the polymer tube, depending on the desired qualities of the guidewire. In some examples, the polymer can be coated afterwards with a PTFE or other lubricious coating to allow efficient catheter/device exchanges. In some examples, by having polymer with sections cut away, there is less contact area with the catheter wall compared to the contact area of a standard guidewire. In this way, in some examples, the force is reduced to track a catheter or other indicated interventional device over a guidewire according to the present subject matter.

In some examples, by twisting a core wire with a unique shape (for example, substantially polygonal such as, but not limited to, hexagonal) and/or forming one or more grooves into a core wire, the tactile feel/bumpy surface can be incorporated into the core wire material. In some examples, the core wire can be coated afterward in a polytetrafluoroethylene (PTFE) coating or other lubricious coating to allow efficient catheter/device exchanges. In some examples, by having a shaped wire (polygonal such as, but not limited to, hexagonal or the like), there is less contact area with the catheter wall. In this way, in some examples, the force is reduced to track a catheter or other indicated interventional device over a guidewire according to the present subject matter.

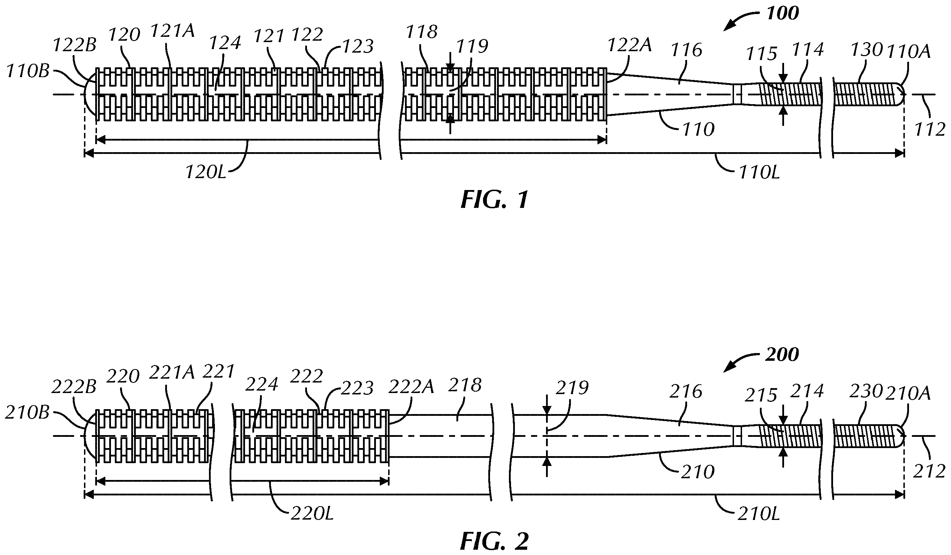

Referring initially to FIGS. 1-5, various examples of guidewires 100, 200, 300, 400, 500 are shown. In some examples, the guidewire 100, 200, 300, 400, 500 includes an elongate core wire 110, 210, 310, 410, 510 including a distal end 110A, 210A, 310A, 410A, 510A, a proximal end 110B, 210B, 310B, 410B, 510B, and a longitudinal axis 112, 212, 312, 412, 512 extending along a length 110L, 210L, 310L, 410L, 510L of the core wire 110, 210, 310, 410, 510 from the distal end 110A, 210A, 310A, 410A, 510A to the proximal end 110B, 210B, 310B, 410B, 510B. In some examples, the guidewire 100, 200, 300, 400, 500 includes a grip portion 120, 220, 320, 420, 520 disposed on the core wire 110, 210, 310, 410, 510. In some examples, the grip portion 120, 220, 320, 420, 520 is disposed proximate the proximal end 110B, 210B, 310B, 410B, 510B of the core wire 110, 210, 310, 410, 510. In some examples, the grip portion 120, 220, 320, 420, 520 is configured to provide tactile feel to a user of the guidewire 100, 200, 300, 400, 500. Tactile feel is used herein to mean a grip, a textured surface, or a surface otherwise configured to allow the user to better hold, grip, and maneuver the guidewire 100, 200, 300, 400, 500; better discern movement longitudinally, rotationally, or otherwise of the guidewire 100, 200, 300, 400, 500 tactilely and/or visually; and or provide tactile feedback to the user while using the guidewire 100, 200, 300, 400, 500. In some examples, the tactile feel of the guidewires 100, 200, 300, 400, 500 described herein allows for better grip of the guidewires 100, 200, 300, 400, 500 as well as giving the user registration of the forward, backward, and/or rotational movement of the guidewires 100, 200, 300, 400, 500 with the changing geometry and/or pattern through the user's hand.

In some examples, the core wire 110, 210, 310, 410, 510 includes a distal portion 114, 214, 314, 414, 514 extending proximally from the distal end 110A, 210A, 310A, 410A, 510A and a proximal portion 118, 218, 318, 418, 518 extending distally from the proximal end 110B, 210B, 310B, 410B, 510B. In some examples, the distal portion 114, 214, 314, 414, 514 includes a distal profile 115, 215, 315, 415, 515 and the proximal portion 118, 218, 318, 418, 518 includes a proximal profile 119, 219, 319, 419, 519. Regardless of what shape the core wire 110, 210, 310, 410, 510 has at the distal portion 114, 214, 314, 414, 514 and the proximal portion 118, 218, 318, 418, 518, the term profile is used to mean the overall size of the core wire 110, 210, 310, 410, 510 at the corresponding portion, whether best measured or described using diameter, thickness, width, cross-sectional area, etc. In some examples, the distal profile 115, 215, 315, 415, 515 is different from the proximal profile 119, 219, 319, 419, 519. In some further examples, the distal profile 115, 215, 315, 415, 515 is smaller than the proximal profile 119, 219, 319, 419, 519. Although such a configuration is shown in FIGS. 1-5, it should be understood that it is within the scope of the present inventive subject matter that the distal profile and the proximal profile be substantially the same or that the distal profile is larger than the proximal profile should such configurations be beneficial for particular applications of the inventive subject matter.

In some examples, wherein the distal profile 115, 215, 315, 415, 515 is different from the proximal profile 119, 219, 319, 419, 519, the core wire 110, 210, 310, 410, 510 can include a transition portion 116, 216, 316, 416, 516 between the distal portion 114, 214, 314, 414, 514 and the proximal portion 118, 218, 318, 418, 518 to transition from the distal profile 115, 215, 315, 415, 515 to the proximal profile 119, 219, 319, 419, 519. In the examples shown in FIGS. 1-5, the transition portion 116, 216, 316, 416, 516 is tapered between the distal profile 115, 215, 315, 415, 515 and the proximal profile 119, 219, 319, 419, 519. However, it is contemplated that the transition portion can take other forms including two or more tapers, one or more stepped transitions, or the like.

The grip portion 120, 220, 320, 420, 520, in some examples, extends at least partially along the proximal portion 118, 218, 318, 418, 518 of the core wire 110, 210, 310, 410, 510. In some examples, the grip portion 120, 320, 420, 520 includes a grip portion length 120L, 320L, 420L, 520L that extends along substantially an entirety of the proximal portion 118, 318, 418, 518. In other examples, the grip portion 220 includes a grip portion length 220L that extends along only a portion of the proximal portion 218. Although the grip portion 120, 320, 420, 520 is shown in FIGS. 1 and 3-5 extending along substantially an entirety of the proximal portion 118, 318, 418, 518, in other examples, the grip portion 120, 320, 420, 520 can extend along only a portion of the proximal portion 118, 318, 418, 518.

In some examples, the guidewire 100, 200, 300, 400, 500 includes a coil 130, 230, 330, 430, 530 disposed at least partially along the distal portion 114, 214, 314, 414, 514. In some examples, the coil 130, 230, 330, 430, 530 is attached to the distal portion 114, 214, 314, 414, 514 of the core wire 110, 210, 310, 410, 510. In some examples, the use of the coil 130, 230, 330, 430, 530 can increase tactile feedback to the user during use of the guidewire 100, 200, 300, 400, 500. It should be understood, however, that, in other examples, the guidewire need not include a coil, depending upon the application for which the guidewire is to be used. For instance, a decreased distal profile and/or deceased friction can be desirable to increase navigability through tortuous vessels. In this instance, a guidewire without a coil can be desirable.

Referring now to FIGS. 1 and 2, in some examples, the grip portion 120, 220 includes a polymer tube 122, 222 applied to the core wire 110, 210. The examples of guidewires 100, 200 shown in FIGS. 1 and 2 are largely similar to one another, differing primarily in the grip portion length 120L, 220L. That is, the example of the guidewire 100 shown in FIG. 1 includes the grip portion 120 having the grip portion length 120L that extends substantially along the entire proximal portion 118 of the core wire 110, whereas the example of the guidewire 200 shown in FIG. 2 includes the grip portion 220 having the grip portion length 220L that extends only partially along the proximal portion 218 of the core wire 210. In various examples, it is contemplated that the grip portion can extend various lengths along the proximal portion of the core wire depending upon the application for the guidewire and the tactile feel desired for the guidewire.

In some examples, the polymer tube 122, 222 includes a sidewall 123, 223 extending between a first end 122A, 222A and a second end 122B, 222B of the polymer tube 122, 222 and at least one cutout 124, 224 through the sidewall 123, 223 of the polymer tube 122, 222. In some examples, the at least one cutout 124, 224 is cut completely through a thickness of the sidewall 123, 223 of the polymer tube 122, 222. In other examples, the at least one cutout 124, 224 is cut partially through the thickness of the sidewall 123, 223 of the polymer tube 122, 222. In some examples, the at least one cutout 124, 224 includes a plurality of cutouts 124, 224 disposed within the sidewall 123, 223 of the polymer tube 122, 222 to form a cut pattern 121, 221 of the grip portion 120, 220. In some examples, the at least one cutout 124, 224 includes at least one laser cut cutout 124, 224 through the sidewall 123, 223 of the polymer tube 122, 222. In other examples, the at least one cutout 124, 224 includes at least one cutout 124, 224 formed through the sidewall 123, 223 of the polymer tube 122, 222 by a mechanical cutting or forming method. The cut patterns 121, 221 shown in FIGS. 1 and 2 include a particular configuration including the cutouts 124, 224 within the sidewall 123, 223 and bridging sections 121A, 221A that form continuous rings around the polymer tube 122, 222 to help the polymer tube 122, 222 maintain its shape. However, this is but one example of many cut patterns that can be implemented on guidewires according to the present inventive subject matter. As such, in other examples, other cut patterns are contemplated herein.

In some examples, the one or more cutouts 124, 224 are cut into the sidewall 123, 223 of the polymer tube 122, 222 prior to application of the polymer tube 122, 222 onto the core wire 110, 210. In some examples, the polymer tube 122, 222 includes a heat shrinkable polymer tube 122, 222, such that the polymer tube 122, 222 can be applied to the core wire 110, 210 by placing the polymer tube 122, 222 in position on the core wire 110, 210 and heating the polymer tube 122, 222 to shrink the polymer tube 122, 222 down around the core wire 110, 210 and attach the polymer tube 122, 222 to the core wire 110, 210.

In other examples, the polymer tube 122, 222 can be attached to the core wire 110, 210 prior to forming of the cut pattern 121, 221 and then the cut pattern 121, 221 can be formed into the polymer tube 122, 222 thereafter. In some examples, the cut pattern 121, 221 can be laser cut into the polymer tube 122, 222 after attachment of the polymer tube 122, 222 to the core wire 110, 210. In other examples, the cut pattern 121, 221 can be cut into the polymer tube 122, 222 using a mechanical cutting method after attachment of the polymer tube 122, 222 to the core wire 110, 210. In still other examples, the cut pattern 121, 221 can be formed into the polymer tube 122, 222 using another mechanical forming method after attachment of the polymer tube 122, 222 to the core wire 110, 210. For instance, in some examples, the cut pattern 121, 221 can be formed into the polymer tube 122, 222 using a hot roller to press the cut pattern 121, 221 into the polymer tube 122, 222 after attachment of the polymer tube 122, 222 to the core wire 110, 210. In still other examples, other methods of forming the cut pattern 121, 221 into the polymer tube 122, 222 are contemplated.

In other examples, it is contemplated that a cut pattern is formed directly into the core wire and the polymer tube is then applied to the core wire over the cut pattern to form a guidewire with tactile feel. Various methods of forming the cut pattern in the core wire are contemplated, such as, but not limited to, machining, laser cutting, stamping, or the like.

In some examples, the tactile feel of the guidewire 100, 200 can be adjusted by increasing or decreasing the number of cutouts 124, 224 per unit length or the length of cutouts 124, 224 per unit length in the polymer tube 122, 222. In some examples, the polymer tube 122, 222 can be used to ensure that the outside diameter of the guidewire 100, 200 is in contact with the catheter or other interventional device. The one or more cutouts 124, 224 of the polymer tube 122, 222, in various examples, allow for a reduced contact area between the catheter or other interventional device and the guidewire 100, 200 to allow for smoother catheter/device exchanges. In some examples, the ease of exchange can be altered by increasing or decreasing the number, the size, and/or the pattern of the cutouts 124, 224 per unit length of the guidewire 100, 200. In various examples, the polymer tube 122, 222 can be cut using a laser cutter or other methods. The polymer tube 122, 222, in various examples, can be formed from various materials including, but not limited to, one or more of PTFE, Estane, or the like.

In some examples, by heat shrinking or otherwise attaching the polymer tube 122, 222 with the cut pattern 121, 221 onto an existing core wire 110, 210, the tactile feel/bumpy surface can be incorporated into existing guidewire designs. The polymer tube 122, 222, in some examples, can be coated afterward in a PTFE or other lubricious coating to allow for efficient catheter/device exchanges. In some examples, the polymer tube 122, 222 with one or more cutouts 124, 224 to form the cut pattern 121, 221 results in less contact area with, for instance, a catheter wall. In this way, the force to track a catheter or other indicated interventional device over the guidewire 100, 200 can be reduced.

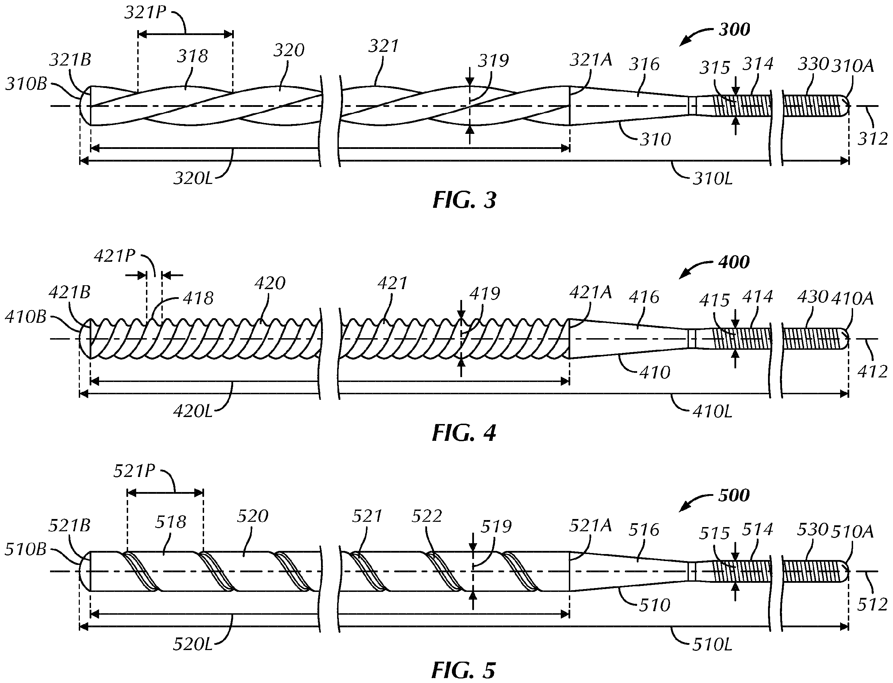

Referring now to FIGS. 3 and 4, in some examples, the grip portion 320, 420 includes a grip pattern 321, 421 formed in the core wire 310, 410. The examples of guidewires 300, 400 shown in FIGS. 3 and 4 are largely similar to one another, differing primarily in a pitch 321P, 421P. As used herein, the term pitch 321P, 421P is meant to mean twists per unit length of the guidewire 300, 400. The example of the guidewire 300 shown in FIG. 3 includes the grip pattern 321 having the pitch 321P that is different than the pitch 421P of the grip pattern 421 of the example of the guidewire 400 shown in FIG. 4. Specifically, in the examples shown, the pitch 321P of the guidewire 300 is less than the pitch 421P of the guidewire 400. These are but two examples of many that can be implemented on guidewires according to the present inventive subject matter. As such, in other examples, other pitches are contemplated herein, such that the pitch can be greater or less than the pitch 321P and/or the pitch 421P shown and described herein depending upon the application and/or desired tactile feel for the guidewire. Additionally, although the grip portion 320, 420 of the guidewire 300, 400 is shown extending the grip portion length 320L, 420L (as measured between a first end 321A, 421A of the grip pattern 321, 421 and a second end 321B, 421B of the grip pattern 321, 421) along substantially the entire proximal portion 318, 418 of the core wire 310, 410, in various other examples, it is contemplated that the grip portion can extend along the proximal portion of the core wire at various lengths other than substantially along the entire proximal portion of the core wire depending upon the application for the guidewire and the tactile feel desired for the guidewire.

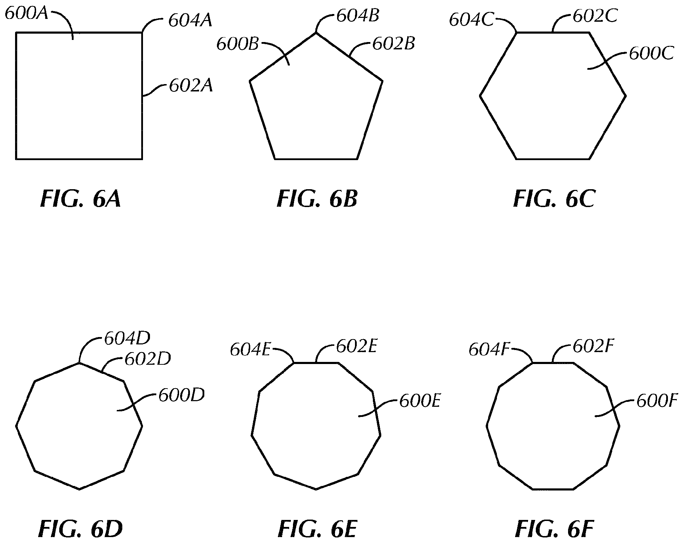

In some examples, the grip pattern 321, 421 includes at least part of the grip portion 320, 420 of the core wire 310, 410 including a non-circular shape 600, 700, 800, 900 in cross section. Referring briefly to FIGS. 6A-9E, various examples of the non-circular shapes 600, 700, 800, 900 are shown. For instance, various examples of polygonal shapes 600A, 600B, 600C, 600D, 600E, 600F are shown in FIGS. 6A-6F, each polygonal shape 600A, 600B, 600C, 600D, 600E, 600F including sides 602A, 602B, 602C, 602D, 602E, 602F meeting at corners 604A, 604B, 604C, 604D, 604E, 604F. Although some polygonal shapes are shown in FIGS. 6A-6F, this is not intended to be all inclusive. As such, polygonal shapes in addition to those shown in FIGS. 6A-6F are contemplated herein. Moreover, although each of the polygonal shapes 600A, 600B, 600C, 600D, 600E, 600F shown include equilateral polygons, it is contemplated that the polygonal shapes include sides of different lengths to one another and corners having different angles to one another.

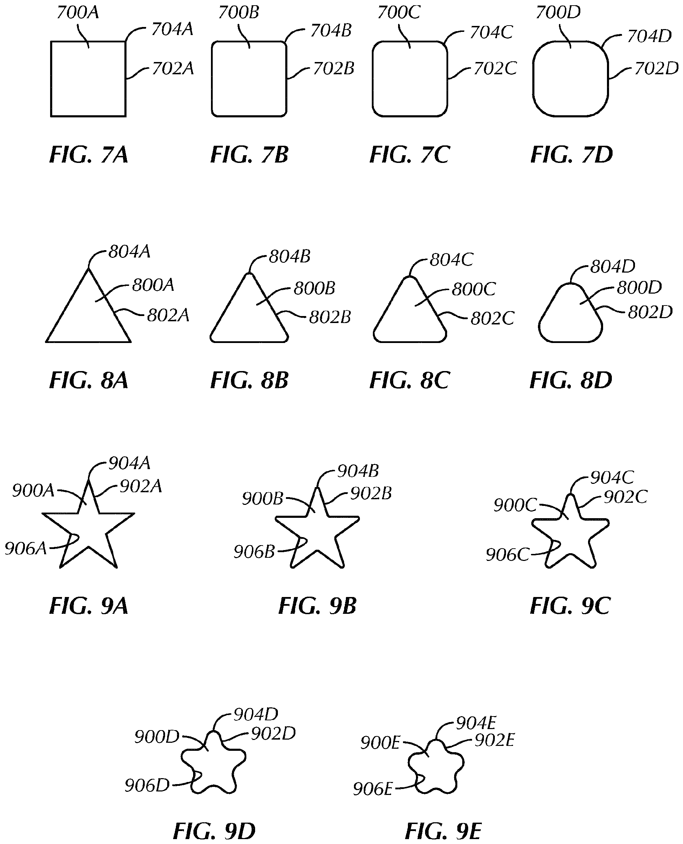

Referring now to FIGS. 7A-9E, various examples of shapes 700, 800, 900 are shown with varying rounding of corners 704, 804, 904, 906 disposed between sides 702, 802, 902. Referring specifically to FIGS. 7A-7D, a rectangle (specifically, a square) shape 700A, 700B, 700C, 700D is shown that is similar to the polygonal shape 600A of FIG. 6A. In some examples, the square shape 700A, 700B, 700C, 700D includes increasing rounding of corners 704A, 704B, 704C, 704D between sides 702A, 702B, 702C, 702D, going from the lesser or no rounding of the corners 704A of FIG. 7A to the greater rounding of the corners 704D of FIG. 7D.

Referring now to FIGS. 8A-8D, a triangle shape 800A, 800B, 800C, 800D is shown. In some examples, the triangle shape 800A, 800B, 800C, 800D includes increasing rounding of corners 804A, 804B, 804C, 804D between sides 802A, 802B, 802C, 802D, going from the lesser or no rounding of the corners 804A of FIG. 8A to the greater rounding of the corners 804D of FIG. 8D.

Referring now to FIGS. 9A-9E, in some examples, the shape of the grip portion 320, 420 can include a star shape 900A, 900B, 900C, 900D, 900E. In some examples, the star shape 900A, 900B, 900C, 900D, 900E includes increasing rounding of outer corners 904A, 904B, 904C, 904D, 904E and inner corners 906A, 906B, 906C, 906D, 906E between sides 902A, 902B, 902C, 902D, 902E, going from the lesser or no rounding of the corners 904A, 906A of FIG. 9A to the greater rounding of the corners 904E, 906E of FIG. 9E.

Although some shapes are shown in FIGS. 7A-9E, this is not intended to be all inclusive. As such, shapes in addition to those shown in FIGS. 7A-9E are contemplated herein. Additionally, although polygonal shapes are shown in FIGS. 7A-9E, it is contemplated that other shapes can be used for the grip portion 320, 420, such as, but not limited to elliptical, oval or egg-shaped, or the like. As such, in various examples, various shapes, as well as various amounts of rounding of corners of the shapes, of the grip portion 320, 420 are contemplated herein.

Referring again to FIGS. 3 and 4, in some examples, at least the part of the grip portion 320, 420 of the core wire 310, 410 is twisted about the longitudinal axis 312, 412 of the core wire 310, 410 to rotate the non-circular shape 600, 700, 800, 900 along at least the part of the grip portion 320, 420. In this way, in some examples, the various facets (such as the corners 604, 704, 804, 904, 906 and the sides 602, 702, 802, 902) of the shape 600, 700, 800, 900 of the grip portion 320, 420 are twisted about the longitudinal axis 312, 412 along at least a portion of the proximal portion 318, 418 of the core wire 310, 410 to create the grip pattern 321, 421 and, in turn, the tactile feel for the guidewire 300, 400. In some examples, the shape 600, 700, 800, 900 of the grip portion 320, 420 is formed by drawing the core wire 310, 410 at least partially through a die with the desired shape. In other examples, the shape 600, 700, 800, 900 of the grip portion 320, 420 can be formed by other mechanical methods such as, but not limited to, machining of the core wire 310, 410 to create the desired shape of the grip portion 320, 420. In some examples, the shape 600, 700, 800, 900 is formed in the grip portion 320, 420 of the core wire 310, 410 by drawing at least a portion of the core wire 310, 410 through the die and twisting the at least the portion of the core wire 310, 410 while drawing to create an uneven surface of at least the portion of the core wire 310, 410, resulting in the tactile feel or grip on the core wire 310, 410. In some examples, after shaping of the grip portion 320, 420 of the core wire 310, 410, the core wire 310, 410 can still be processed as per a standard guidewire and/or ground or otherwise processed to create the distal portion 314, 414 or tip.

In some examples, by drawing the shape 600, 700, 800, 900 and twisting the pitch 321P, 421P into the core wire 310, 410, the need for additional processing steps to create the tactile feel is reduced, if not eliminated. In some examples, the twisted core wire 310, 410 can be coated (for instance, with PTFE) and maintain tactile feel. The tactile feel, in some examples, can be adjusted by adjusting the pitch 321P, 421P of the grip pattern 321, 421 (for instance, increasing or decreasing the number of twists per unit length of the grip portion 320, 420) and/or changing the die shape or machining or otherwise forming the core wire 310, 410 differently to change the shape 600, 700, 800, 900 of the core wire 310, 410 at least at the grip portion 320, 420. In some examples, the twist of the core wire 310, 410 can ensure that an outer diameter of the guidewire 300, 400 is in contact with the catheter or other interventional device, while, at the same time, a reduced contact area between the interventional device and the guidewire 300, 400 can allow for smoother catheter/device exchanges. In some examples, the pitch 321P, 421P can be altered to reduce or increase the contact area. That is, the lower the pitch 321P, 421P, the less contact area and the lower the force needed to track the catheter or other interventional device over the guidewire 300, 400, and, in turn, the higher the pitch 321P, 421P, the more contact area and the higher the force needed to track the catheter or other interventional device over the guidewire 300, 400.

Referring now to FIG. 5, in some examples, the grip portion 520 includes a grip pattern 521 formed in the core wire 510. In some examples, the grip pattern 521 includes at least one groove 522 cut or otherwise formed into the core wire 510. In some examples, the groove 522 extends along at least part of the grip portion 520 of the core wire 510. The at least one groove 522, in some examples, is substantially helically or spirally extends along the core wire 510. For instance, in some examples, the groove 522 substantially rotates around the longitudinal axis 512 of the core wire 510 as the groove 522 extends along at least a portion of the grip portion length 520L (as measured between a first end 521A of the grip pattern 521 and a second end 521B of the grip pattern 521) of the core wire 510. Although shown with one groove 510 formed in the core wire 510 in FIG. 5, it is further contemplated herein that the core wire includes two or more grooves formed in the core wire depending upon the application for which the guidewire is to be used. Moreover, in various examples, a shape of the groove 522 and/or a path of the groove 522 along the core wire 510 can be varied depending upon the application for which the guidewire 500 is to be used and/or the desired tactile feel for the guidewire 500. In various examples, the shape of the groove 522 (in cross section) can include a U-shape (with sharp corners, rounded corners, or a continuous curve), a V-shape, or other shapes.

The groove 522 of the grip pattern 521 of the guidewire 500 includes a pitch 521P. As used herein, the term pitch 521P is meant to mean rotations of the groove 500 around the core wire 510 per unit length of the guidewire 500. Although the example of the guidewire 500 shown in FIG. 5 includes the grip pattern 521 having a particular pitch 521P, this is but one example of many pitches that can be implemented on guidewires according to the present inventive subject matter. As such, in other examples, other pitches are contemplated herein, such that the pitch can be greater or less than the pitch 521P shown and described herein depending upon the application and/or desired tactile feel for the guidewire. Additionally, although the grip portion 520 of the guidewire 500 is shown extending the grip portion length 520L along substantially the entire proximal portion 518 of the core wire 510, in various other examples, it is contemplated that the grip portion can extend along the proximal portion of the core wire at various lengths other than substantially along the entire proximal portion of the core wire depending upon the application for the guidewire and the tactile feel desired for the guidewire.

In various examples, the groove 522 can be formed by various mechanical methods, such as, but not limited to, machining of the core wire 510 to create the desired groove 522 of the grip portion 520. In other examples, the groove 522 522 can be formed by other mechanical methods, such as stamping. In still other examples, 522 can be formed by various non-mechanical methods, such as laser profiling, thermal processes, or energy processes. In some examples, after forming of the groove 522 of the grip portion 520 of the core wire 510, the core wire 510 can still be processed as per a standard guidewire and/or ground or otherwise processed to create the distal portion 514 or tip. In some examples, by forming the groove 522 at the desired pitch 521P into the core wire 510, the need for additional processing steps to create the tactile feel is reduced, if not eliminated. In some examples, the core wire 510 can be coated (for instance, with PTFE) and maintain tactile feel. The tactile feel, in some examples, can be adjusted by adjusting the pitch 521P of the groove 522 of the grip pattern 521 (for instance, increasing or decreasing the number of rotations of the groove 522 per unit length of the grip portion 520).

In some examples, the groove 522 of the core wire 510 can ensure that an outer diameter of the guidewire 500 is in contact with the catheter or other interventional device, while, at the same time, a reduced contact area between the interventional device and the guidewire 500 can allow for smoother catheter/device exchanges. In some examples, the pitch 521P can be altered to reduce or increase the contact area. That is, the lower the pitch 521P, the less contact area and the lower the force needed to track the catheter or other interventional device over the guidewire 500, and, in turn, the higher the pitch 521P, the more contact area and the higher the force needed to track the catheter or other interventional device over the guidewire 500.

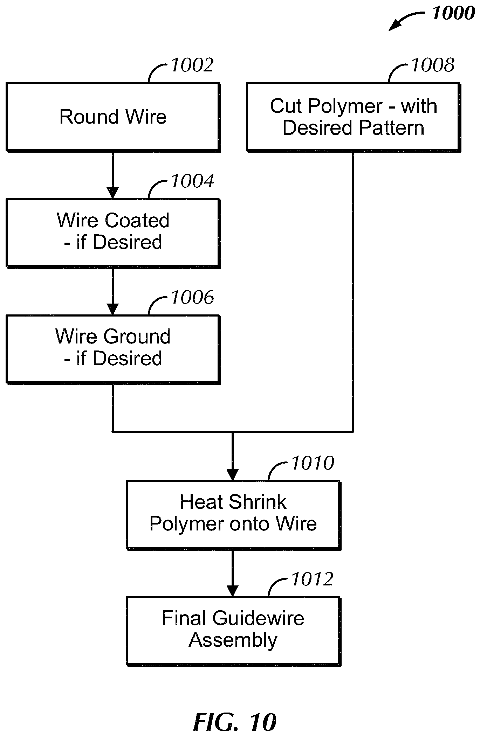

Referring to FIG. 10, with further reference to FIGS. 1 and 2 and the accompanying description above, a method 1000 of making the guidewire 100, 200 with tactile feel is shown. The core wire 110, 210 is provided 1002, the core wire 110, 210 including a substantially round cross-sectional shape. The core wire 110, 210 can be coated 1004 (with PTFE or another coating, for instance) and/or ground down 1006 (for instance to form the distal portion 114, 214; the transition portion 116, 216; and/or the proximal portion 118, 218). In some examples, the core wire 110, 210 can be coated 1004 before and/or after being ground down 1006. In this way, in some examples, the elongate core wire 110, 210 is formed, with the core wire 110, 210 including the distal end 110A, 210A; the proximal end 110B, 210B;
and the longitudinal axis 112, 212 extending along the length 110L, 210L of the core wire 110, 210 from the distal end 110A, 210A to the proximal end 110B, 210B. The grip portion 120, 220 is then formed on the core wire 110, 210, the grip portion 120, 220 being configured to provide tactile feel to the user of the guidewire 100, 200. In some examples, the grip portion 120, 220 is formed on the core wire 110, 210 proximate the proximal end 118, 218 of the core wire 110, 210.

Forming the grip portion 120, 220 includes cutting 1008 the polymer tube 122, 222 with the desired cut pattern 121, 221. This includes cutting the at least one cutout 124, 224 through the sidewall 123, 223 of the polymer tube 122, 222. In some examples, the at least one cutout 124, 224 is cut completely through a thickness of the sidewall 123, 223 of the polymer tube 122, 222. In other examples, the at least one cutout 124, 224 is cut partially through the thickness of the sidewall 123, 223 of the polymer tube 122, 222. The polymer tube 122, 222 includes the sidewall 123, 223 extending between the first end 122A, 222A and the second end 122B, 222B of the polymer tube 122, 222. In some examples, the at least one cutout 124, 224 forms the cut pattern 121, 221 of the grip portion 120, 220. In further examples, a plurality of cutouts 124, 224 disposed within the sidewall 123, 223 of the polymer tube 122, 222 forms the cut pattern 121, 221 of the grip portion 120, 220. In some examples, the one or more cutouts 124, 224 of the cut pattern 121, 221 are laser cut in the polymer tube 122, 222. In other examples, the one or more cutouts 124, 224 of the cut pattern 121, 221 are otherwise formed into the polymer tube 122, 222, including, but not limited to, being mechanically cut or machined into the polymer tube 122, 222, being formed into the polymer tube 122, 222 by rolling a hot roller over the polymer tube 122, 222, or the like.

The polymer tube 122, 222 is be attached to the core wire 110, 210. In some examples, the polymer tube 122, 222 is attached by heat shrinking 1010 the polymer tube 122, 222 to the core wire 110, 210. In some examples, the cut pattern 121, 221 is formed into the polymer tube 122, 222 prior to attachment of the polymer tube 122, 222 to the core wire 110, 210. In other examples, the cut pattern 121, 221 is formed into the polymer tube 122, 222 after attachment of the polymer tube 122, 222 to the core wire 110, 210.

Thereafter, in some examples, final guidewire assembly 1012 occurs during which various aspects of the guidewire 100, 200 are finalized. Such aspects include, but are not limited to, one or more coatings being applied to the guidewire 100, 200; the coil 130, 230 being attached to the distal portion 114, 214 of the core wire 110, 210; jacketing the core wire 110, 210; and/or the like.

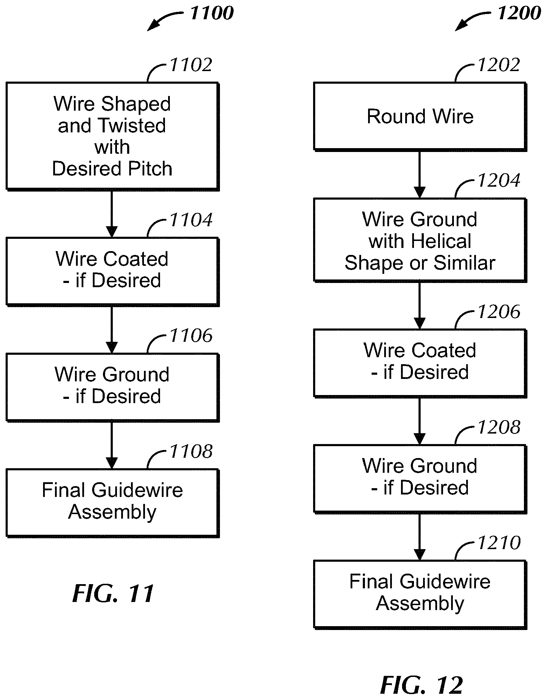

Referring to FIG. 11, with further reference to FIGS. 3 and 4 and the accompanying description above, a method 1100 of making the guidewire 300, 400 with tactile feel is shown. In some examples, the core wire 310, 410 is shaped and twisted 1102. In some examples, the core wire 310, 410 includes at least a portion that includes the grip pattern 321, 421. In some examples, the grip pattern 321, 421 includes a substantially non-circular cross-sectional shape 600, 700, 800, 900 twisted at the desired pitch 321P, 421P to form the grip portion 320, 420. In some examples, the non-circular shape 600, 700, 800, 900 is formed by drawing at least a portion of the core wire 310, 410 through a complementarily-shaped die. In other examples, the non-circular shape 600, 700, 800, 900 can be formed by mechanical methods, such as machining, for instance. In some examples, at least the part of the grip portion 320, 420 of the core wire 310, 410 is twisted about the longitudinal axis 312, 412 of the core wire 310, 410 to rotate the non-circular shape 600, 700, 800, 900 along at least the part of the grip portion 320, 420.

The core wire 310, 410 can be coated 1104 (with PTFE or another coating, for instance) and/or ground down 1106 (for instance to form the distal portion 314, 414; the transition portion 316, 416; and/or the proximal portion 318, 418). In some examples, the core wire 310, 410 can be coated 1104 before and/or after being ground down 1106. In this way, in some examples, the elongate core wire 310, 410 is formed, with the core wire 310, 410 including the distal end 310A, 410A; the proximal end 310B, 410B; and the longitudinal axis 312, 412 extending along the length 310L, 410L of the core wire 310, 410 from the distal end 310A, 410A to the proximal end 310B, 410B. In some examples, the grip portion 320, 420 formed on the core wire 310, 410 is configured to provide tactile feel to the user of the guidewire 300, 400. In some examples, the grip portion 320, 420 is formed on the core wire 310, 410 proximate the proximal end 318, 418 of the core wire 310, 410.

Thereafter, in some examples, final guidewire assembly 1108 occurs during which various aspects of the guidewire 300, 400 are finalized. Such aspects include, but are not limited to, one or more coatings being applied to the guidewire 300, 400; the coil 330, 430 being attached to the distal portion 314, 414 of the core wire 310, 410; jacketing the core wire 310, 410; and/or the like.

Referring to FIG. 12, with further reference to FIG. 5 and the accompanying description above, a method 1200 of making the guidewire 500 with tactile feel is shown. In some examples, the core wire 510 is provided 1202, the core wire 510 including a substantially round cross-sectional shape. In some examples, the grip portion 520 is then formed 1204 on the core wire 510, the grip portion 520 being configured to provide tactile feel to the user of the guidewire 500. In some examples, the grip portion 520 is formed on the core wire 510 proximate the proximal end 518 of the core wire 510. In some examples, the core wire 510 includes at least a portion that includes the grip pattern 521. In some examples, the grip pattern 521 is formed in the core wire 510 by cutting the at least one groove 522 into the core wire 510, the groove 522 extending along at least part of the grip portion 520 of the core wire 510. In some examples, the at least one groove 522 is helically or spirally cut into the core wire 510 so that the at least one groove 522 helically or spirally extends along the core wire 510.

The core wire 510 can be coated 1206 (with PTFE or another coating, for instance) and/or ground down 1208 (for instance to form the distal portion 514; the transition portion 516; and/or the proximal portion 518). In some examples, the core wire 510 can be coated 1206 before and/or after being ground down 1208. In this way, in some examples, the elongate core wire 510 is formed, with the core wire 510 including the distal end 510A; the proximal end 510B; and the longitudinal axis 512 extending along the length 510L of the core wire 510 from the distal end 510A to the proximal end 510B.

Thereafter, in some examples, final guidewire assembly 1210 occurs during which various aspects of the guidewire 500 are finalized. Such aspects include, but are not limited to, one or more coatings being applied to the guidewire 500; the coil 530 being attached to the distal portion 514 of the core wire 510; jacketing the core wire 510; and/or the like.

The present inventors have recognized various advantages of the subject matter described herein. The present inventors have recognized, among other things, that the present subject matter can be used to provide a guidewire with tactile feel to satisfy design requirements required by physicians by incorporating the tactile feel into the guidewire. In various examples, the present subject matter is advantageous in that it provides for a guidewire that includes a grip portion to enhance tactile feel, handling, control, and advancement recognition for the physician or other user of the guidewire. In some examples, the present invention facilitates tracking of a catheter or other indicated interventional device over the guidewire by reducing a force needed to do so. While various advantages of the example systems are listed herein, this list is not considered to be complete, as further advantages may become apparent from the description and figures presented herein.

Although the subject matter of the present patent application has been described with reference to various examples, workers skilled in the art will recognize that changes can be made in form and detail without departing from the scope defined by the claims.

The above Detailed Description includes references to the accompanying drawings. The drawings show, by way of illustration, specific examples in which the present apparatuses and methods can be practiced. These embodiments are also referred to herein as "examples."

In this document, the terms "a" or "an" are used to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "about" and "approximately" or similar are used to refer to an amount that is nearly, almost, or in the vicinity of being equal to a stated amount.

In the appended claims, the terms "including" and "in which" are used as the plain-English equivalents of the respective terms "comprising" and "wherein." Also, in the following claims, the terms "including" and "comprising" are open-ended, that is, an apparatus or method that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

## Claims

1. A guidewire (100) comprising:
an elongate core wire (110) including a distal end (110A), a proximal end (110B), and a longitudinal axis (112) extending along a length (110L) of the core wire from the distal end to the proximal end; and
a grip portion (120) disposed on the core wire, the grip portion configured to provide tactile feel to a user of the guidewire;
**characterized in that** the grip portion includes a polymer tube (122) applied to the core wire, the polymer tube (122) including a sidewall (123) extending between first (122A) and second (122B) ends of the polymer tube and at least one cutout (124) through the sidewall of the polymer tube.

2. The guidewire of claim 1, wherein the core wire (110) includes a distal portion (114) extending proximally from the distal end and a proximal portion (118) extending distally from the proximal end, the distal portion including a distal profile (115) and the proximal portion including a proximal profile (119), the distal profile being smaller than the proximal profile.

3. The guidewire of claim 2, wherein the grip portion (120) extends at least partially along the proximal portion of the core wire.

4. The guidewire of any one of the preceding claims, wherein the at least one cutout (124) includes a plurality of cutouts disposed within the sidewall of the polymer tube to form a cut pattern of the grip portion.

5. The guidewire of any one of the preceding claims, wherein the at least one cutout (124) includes at least one laser cut cutout through the sidewall of the polymer tube.

6. The guidewire of any one of the preceding claims, wherein the polymer tube (122) includes a heat shrinkable polymer tube.

7. The guidewire of any one of the preceding claims, wherein the grip portion (120) includes a grip pattern (321) formed in the core wire.

8. The guidewire of claim 7, wherein the grip pattern (321) includes at least one groove cut into the core wire, the groove extending along at least part of the grip portion of the core wire.

9. The guidewire of claim 8, wherein the at least one groove (522) spirally extends along the core wire.

10. The guidewire of claim 7 or claim 8, wherein the grip pattern includes at least part of the grip portion of the core wire including a non-circular shape, at least the part of the grip portion of the core wire being twisted about the longitudinal axis of the core wire to rotate the non-circular shape along at least the part of the grip portion.

11. A method of making a guidewire with tactile feel, the method comprising:
forming an elongate core wire (110), the core wire including a distal end, a proximal end, and a longitudinal axis extending along a length of the core wire from the distal end to the proximal end; and
forming a grip portion (120) on the core wire, the grip portion configured to provide tactile feel to a user of the guidewire;
wherein forming the grip portion includes:
cutting at least one cutout (124) through a sidewall of a polymer tube (122), the polymer tube including the sidewall extending between first and second ends of the polymer tube; and
applying the polymer tube to the core wire, wherein the at least one cutout forms a cut pattern of the grip portion.

12. The method of claim 11, wherein applying the polymer tube includes heat shrinking the polymer tube onto the core wire.

13. The method of claim 11 or claim 12, wherein forming the grip portion includes forming a grip pattern in the core wire including cutting at least one groove into the core wire, the groove extending along at least part of the grip portion of the core wire.

14. The method of claim 13, wherein cutting the at least one groove into the core wire includes spirally cutting the at least one groove into the core wire so that the at least one groove spirally extends along the core wire.

15. The method of any of claims 11 to 14, wherein forming the grip portion includes forming a grip pattern in the core wire including:
forming at least part of the grip portion of the core wire into a non-circular shape; and
twisting at least the part of the grip portion of the core wire about the longitudinal axis of the core wire to rotate the non-circular shape along at least the part of the grip portion.

## Patentansprüche

1. Führungsdraht (100), der Folgendes umfasst:
einen länglichen Kerndraht (110) mit einem distalen Ende (110A), einem proximalen Ende (110B) und einer Längsachse (112), die sich entlang einer Länge (110L) des Kerndrahts von dem distalen Ende zu dem proximalen Ende erstreckt; und
einen Griffabschnitt (120), der an dem Kerndraht angeordnet ist, wobei der Griffabschnitt dafür konfiguriert ist, einem Benutzer des Führungsdrahts ein taktiles Gefühl zu vermitteln;
**dadurch gekennzeichnet, dass** der Griffabschnitt einen Polymerschlauch (122) umfasst, der auf den Kerndraht aufgebracht ist, wobei der Polymerschlauch (122) eine Seitenwand (123), die sich zwischen einem ersten (122A) und einem zweiten (122B) Ende des Polymerschlauches erstreckt, und mindestens einen Ausschnitt (124) durch die Seitenwand des Polymerschlauches umfasst.

2. Führungsdraht nach Anspruch 1, wobei der Kerndraht (110) einen distalen Abschnitt (114), der sich proximal von dem distalen Ende erstreckt, und einen proximalen Abschnitt (118), der sich distal von dem proximalen Ende erstreckt, umfasst, wobei der distale Abschnitt ein distales Profil (115) umfasst und der proximale Abschnitt ein proximales Profil (119) umfasst, wobei das distale Profil kleiner ist als das proximale Profil.

3. Führungsdraht nach Anspruch 2, wobei sich der Griffabschnitt (120) mindestens teilweise entlang des proximalen Abschnitts des Kerndrahts erstreckt.

4. Führungsdraht nach einem der vorhergehenden Ansprüche, wobei die mindestens eine Aussparung (124) eine Vielzahl von Aussparungen umfasst, die innerhalb der Seitenwand des Polymerrohrs angeordnet sind, um ein Schnittmuster des Griffabschnitts zu formen.

5. Führungsdraht nach einem der vorhergehenden Ansprüche, wobei der mindestens eine Ausschnitt (124) mindestens einen Laserausschnitt durch die Seitenwand des Polymerrohrs aufweist.

6. Führungsdraht nach einem der vorhergehenden Ansprüche, wobei der Polymerschlauch (122) einen wärmeschrumpfenden Polymerschlauch umfasst.

7. Führungsdraht nach einem der vorhergehenden Ansprüche, wobei der Griffabschnitt (120) ein im Kerndraht geformtes Griffmuster (321) umfasst.

8. Führungsdraht nach Anspruch 7, wobei das Griffmuster (321) mindestens eine in den Kerndraht geschnittene Rille umfasst, wobei sich die Rille mindestens entlang eines Teils des Griffbereichs des Kerndrahts erstreckt.

9. Führungsdraht nach Anspruch 8, wobei sich die mindestens eine Rille (522) spiralförmig entlang des Kerndrahts erstreckt.

10. Führungsdraht nach Anspruch 7 oder Anspruch 8, wobei das Griffmuster mindestens einen Teil des Griffabschnitts des Kerndrahts umfasst, der eine nicht kreisförmige Form umfasst, wobei mindestens der Teil des Griffabschnitts des Kerndrahts um die Längsachse des Kerndrahts verdreht ist, um die nicht kreisförmige Form entlang mindestens des Teils des Griffabschnitts zu drehen.

11. Verfahren zur Herstellung eines Führungsdrahts mit taktilem Gefühl, wobei das Verfahren Folgendes umfasst:
Formen eines länglichen Kerndrahts (110), wobei der Kerndraht ein distales Ende, ein proximales Ende und eine Längsachse umfasst, die sich entlang der Länge des Kerndrahts von dem distalen Ende zu dem proximalen Ende erstreckt; und
Formen eines Griffabschnitts (120) an dem Kerndraht, wobei der Griffabschnitt dafür konfiguriert ist, einem Benutzer des Führungsdrahts ein taktiles Gefühl zu vermitteln;
wobei das Formen des Griffabschnitts Folgendes umfasst:
Schneiden mindestens eines Ausschnitts (124) durch eine Seitenwand eines Polymerschlauchs (122), wobei der Polymerschlauch die Seitenwand umfasst, die sich zwischen einem ersten und einem zweiten Ende des Polymerschlauchs erstreckt; und
Anbringen des Polymerrohrs an den Kerndraht, wobei der mindestens eine Ausschnitt ein Schnittmuster des Griffabschnitts formt.

12. Verfahren nach Anspruch 11, wobei das Aufbringen des Polymerschlauches das Wärmeschrumpfen des Polymerschlauches auf den Kerndraht umfasst.

13. Verfahren nach Anspruch 11 oder Anspruch 12, wobei das Formen des Griffabschnitts das Formen eines Griffmusters in dem Kerndraht umfasst, einschließlich des Einschneidens mindestens einer Rille in den Kerndraht, wobei sich die Rille entlang mindestens eines Teils des Griffabschnitts des Kerndrahts erstreckt.

14. Verfahren nach Anspruch 13, wobei das Schneiden der mindestens einen Rille in den Kerndraht das spiralförmige Schneiden der mindestens einen Rille in den Kerndraht umfasst, so dass sich die mindestens eine Rille spiralförmig entlang des Kerndrahts erstreckt.

15. Verfahren nach einem der Ansprüche 11 bis 14, wobei das Formen des Griffabschnitts das Formen eines Griffmusters in dem Kerndraht umfasst, das Folgendes umfasst:
Formen mindestens eines Teils des Griffabschnitts des Kerndrahts in eine nicht kreisförmige Form; und
Verdrehen mindestens des Teils des Griffabschnitts des Kerndrahts um die Längsachse des Kerndrahts, um die nicht kreisförmige Form entlang mindestens des Teils des Griffabschnitts zu drehen.

## Revendications

1. Fil de guidage (100) comprenant :
un fil central allongé (110) comprenant une extrémité distale (110A), une extrémité proximale (110B) et un axe longitudinal (112) s'étendant le long d'une longueur (110L) du fil central depuis l'extrémité distale à l'extrémité proximale ; et
une partie de préhension (120) disposée sur le fil central, la partie de préhension étant configurée pour procurer une sensation tactile à un utilisateur du fil de guidage ;
**caractérisée en ce que** la partie de préhension comprend un tube polymère (122) appliqué au fil central, le tube polymère (122) comprenant une paroi latérale (123) s'étendant entre des première (122A) et seconde (122B) extrémités du tube polymère et au moins une découpe (124) à travers la paroi latérale du tube polymère.

2. Fil de guidage selon la revendication 1, dans lequel le fil central (110) comprend une partie distale (114) s'étendant de manière proximale à partir de l'extrémité distale et une partie proximale (118) s'étendant de manière distale à partir de l'extrémité proximale, la partie distale comprenant un profil distal (115) et la partie proximale comprenant un profil proximal (119), le profil distal étant plus petit que le profil proximal.

3. Fil de guidage selon la revendication 2, dans lequel la partie de préhension (120) s'étend au moins partiellement le long de la partie proximale du fil central.

4. Fil de guidage selon l'une quelconque des revendications précédentes, dans lequel l'au moins une découpe (124) comprend une pluralité de découpes disposées à l'intérieur de la paroi latérale du tube polymère pour former un motif découpé de la partie de préhension.

5. Fil de guidage selon l'une quelconque des revendications précédentes, dans lequel l'au moins une découpe (124) comprend au moins une découpe découpée au laser à travers la paroi latérale du tube polymère.

6. Fil de guidage selon l'une quelconque des revendications précédentes, dans lequel le tube polymère (122) comprend un tube polymère thermorétractable.

7. Fil de guidage selon l'une quelconque des revendications précédentes, dans lequel la partie de préhension (120) comprend un motif de préhension (321) formé dans le fil central.

8. Fil de guidage selon la revendication 7, dans lequel le motif de préhension (321) comprend au moins une rainure découpée dans le fil central, la rainure s'étendant le long d'au moins une partie de la partie de préhension du fil central.

9. Fil de guidage selon la revendication 8, dans lequel l'au moins une rainure (522) s'étend en spirale le long du fil central.

10. Fil de guidage selon la revendication 7 ou la revendication 8, dans lequel le motif de préhension comprend au moins une partie de la partie de préhension du fil central comprenant une forme non circulaire, au moins la partie de la partie de préhension du fil central étant torsadée autour de l'axe longitudinal du fil central pour faire tourner la forme non circulaire le long d'au moins la partie de la partie de préhension.

11. Procédé de fabrication d'un fil de guidage avec sensation tactile, le procédé comprenant :
la formation d'un fil central allongé (110), le fil central comprenant une extrémité distale, une extrémité proximale et un axe longitudinal s'étendant le long d'une longueur du fil central depuis l'extrémité distale à l'extrémité proximale ; et
la formation d'une partie de préhension (120) sur le fil central, la partie de préhension étant configurée pour fournir une sensation tactile à un utilisateur du fil de guidage ;
dans laquelle la formation de la partie de préhension comprend :
la découpe d'au moins une découpe (124) à travers une paroi latérale d'un tube polymère (122), le tube polymère comprenant la paroi latérale s'étendant entre des première et seconde extrémités du tube polymère ; et
l'application du tube polymère sur le fil central, dans lequel l'au moins une découpe forme un motif de découpe de la partie de préhension.

12. Procédé selon la revendication 11, dans lequel l'application du tube polymère comprend la thermorétraction du tube polymère sur le fil central.

13. Procédé selon la revendication 11 ou la revendication 12, dans lequel la formation de la partie de préhension comprend la formation d'un motif de préhension dans le fil central comprenant la découpe d'au moins une rainure dans le fil central, la rainure s'étendant le long d'au moins une partie de la partie de préhension du fil central.

14. Procédé selon la revendication 13, dans lequel la découpe de l'au moins une rainure dans le fil central comprend la découpe en spirale de l'au moins une rainure dans le fil central de sorte que l'au moins une rainure s'étende en spirale le long du fil central.

15. Procédé selon l'une quelconque des revendications 11 à 14, dans lequel la formation de la partie de préhension comprend la formation d'un motif de préhension dans le fil central comprenant :
la formation d'au moins une partie de la partie de préhension du fil central en une forme non circulaire ; et
la torsion d'au moins la partie de la partie de préhension du fil central autour de l'axe longitudinal du fil central pour faire tourner la forme non circulaire le long d'au moins la partie de la partie de préhension.
